(19) Europäisches Patentamt
European Patent Office
Office européen des brevets

(11) EP 0 438 796 B1

(12) EUROPEAN PATENT SPECIFICATION

(45) Date of publication and mention of the grant of the patent:
26.08.1998 Bulletin 1998/35

(51) Int. Cl.[6]: C07D 209/34, B01J 31/02, C07B 57/00

(21) Application number: 90125644.6

(22) Date of filing: 28.12.1990

(54) **Process for the enantioselection synthesis of alkylated oxindoles used as intermediates in the preparation of physostigmine**

Verfahren zur enantioselektiven Synthese alkylierter Oxindole zur Verwendung als Zwischenprodukte bei der Herstellung von Physostigminen

Procédé pour la synthèse énantiosélective des oxindoles alkylés utilisables comme composés intermédiaires pour la préparation de physostigmine

(84) Designated Contracting States:
**AT BE CH DE DK ES FR GB GR IT LI LU NL SE**

(30) Priority: **22.01.1990 US 469882**

(43) Date of publication of application:
**31.07.1991 Bulletin 1991/31**

(73) Proprietor:
**HOECHST MARION ROUSSEL, Inc.**
**Kansas City, Missouri 64137-1405 (US)**

(72) Inventors:
- **Lee, Thomas Bing Kin, Dr.**
  **Whitehouse Station, NJ 08889 (US)**
- **Wong, George S.K.**
  **Summit, NJ 07901 (US)**

(74) Representative:
**Losert, Wolfgang Dr. et al**
**Hoechst AG**
**Patent- und Lizenzabteilung**
**Gebäude K 801**
**D-65926 Frankfurt am Main (DE)**

(56) References cited:
- **CHEMICAL ABSTRACTS, vol. 111, no. 21, 20 November 1989 Columbus, Ohio, USA BANGHUA CHEN et al.: "Asymmetric C-alkylation with chiral phase-transfer catalysis. Synthesis of optically active derivatives of 2-indolinone" page 748; ref. no. 194508A & Huaxue Xuebao 1989, 47[4], 350-4**
- **JOURNAL OF ORGANIC CHEMISTRY, vol. 52, no. 21, 1987, pages 4745 - 4752; D.L. HUGHES et al.: "Efficient Catalytic Asymmetric Alkylations. 3. A Kinetic and Mechanistic Study of the Enantioselective Phase-Transfer ..."**
- **JOURNAL OF THE AMERICAN CHEMICAL SOCIETY, vol. 111, no. 6, 1989, pages 2353 - 2355; M. J. O'DONNELL et al.: "The Stereoselective Synthesis of -Amino Acids by Phase-Transfer Catalysis"**
- **CHEMICAL ABSTRACTS, vol. 107, no. 23, 07 December 1987 Columbus, Ohio, USA U.H. DOLLING et al.: "Efficient asymmetric alkylations via chiral phase-transfer catalysis: applications and mechanism" page 513; ref. no. 216813T & ACS Symp. Ser. 1987, 326, pages 67-81**
- **CHEMICAL AND PHARMACEUTICAL BULLETIN, vol. 30, no. 7, 1982, pages 2641 - 2643; S. TAKANO et al.: "Enantioselective synthesis of [-]-physostigmine."**
- **Helv. Chim. Acta 69(1986),pp.1486-1497**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.3/3.4

## Description

This invention relates to a process for the stereoselective synthesis of (+)-physostigmine and (-)-physostigmine.

Background of the invention

The cholinergic neuronal system can be found in the central nervous system (CNS), in the autonomic nervous system, and in the skeletal motor system. Acetylcholine (ACh) is the neurotransmitter in all ganglia, the neuromuscular junction, and the post-ganglionic synapses of the cholinergic nervous system. Acetylcholine is normally an excitatory neurotransmitter that binds to nicotinic and muscarinic receptors.

Acetylcholinesterase (AChE) is an enzyme that hydrolyzes and thereby deactivates ACh after it binds to a receptor. This enzyme is present in all peripheral and central junctional sites and in certain cells of the body.

In some circumstances, it is desirable to stimulate acetylcholine receptors. One method involves the use of indirect agonists, such as anticholinesterase drugs, which inhibit the hydrolysis of ACh by AChE. When an anticholinesterase drug blocks AChE and inhibits the destruction of released ACh, a higher neurotransmitter level and increased biological response result. The alkaloid, physostigmine, which can be isolated from the seeds of the Calabar bean, has been found to be particularly effective as an anticholinesterase drug. Physostigmine has a high affinity for AChE and is capable of inhibiting AChE for prolonged periods.

It is believed that degeneration of the cholinergic pathways in the CNS and the resultant development of apparent irregularities in neuron arrangement may be a principle cause of senile dementia of the Alzheimer type. This disease leads to progressive regression of memory and learned functions. Since the average age of the population is on the increase, the frequency of Alzheimer's disease is increasing and requires urgent attention.

It has been suggested that cholinergic agonists, such as the anticholinesterase drugs, are useful in the treatment of Alzheimer's disease. Nevertheless, drug treatment with anticholinesterase drugs has not proved entirely satisfactory. Thus, there is a need in the art for new forms of drugs for the treatment of this disease.

The enantiomers of physostigmine and pharmaceutically active physostigmine-like compounds, such as the compounds described in U.S. Patent 4,791,107, are under investigation for the treatment of Alzheimer's disease. In order to satisfy the need for compounds having the highest pharmaceutical activity, there exists a need in the art for a process for the stereoselective synthesis of the enantiomers. Specifically, the enantiomer (-)-physostigmine is of current interest, and while methods for preparing physostigmine and physostigmine-like compounds have been proposed, there exists a need in the art for a stereoselective process for producing the S- or (-)-forms.

Processes for the selective synthesis of stereoisomers by catalytic asymmetric alkylation are described in documents (1) to (4) below:

(1) Chem. Abs. 111 (1989), 1945089 discloses the asymmetric C-alkylation of 1,3-dimethyl-5-methoxy-2-indoline with alkyl bromide and a chiral quaternary ammonium salt as a phase-transfer catalyst.

(2) J. Org. chem. 1987, 52, 4745-4752 discloses the phase-transfer asymmetric methylation of 6,7-dichloro-5-methoxy-2-phenyl-1-indanone using methyl halide and a substituted N-benzyl-cinchoninium halide.

(3) J. Amer. Chem. soc. 1989, 1989, 111, 2353-2355 discloses the asymmetric synthesis of α-amino acids by phase-transfer catalytic alkylation using a chiral catalyst derived from cinchona alkalides and a prochiral protected glycine derivative.

(4) Phys. Org. Chem. 107, 1987, 216813t discloses the catalytic asymmetric methylation of 6,7-dichloro-5-methoxy-2-phenyl-1-indanone with methylchloride using N-substituted circhonium bromide as chiral phase transfer catalyst.

Chem. Pharm. Bull. 30 (1982), 2641-2643, discloses a process to prepare (-)-physostigmine starting from (s)-(-)-benzyl-2,3-epoxypropyl ether.

It has been found that the compound 1,3-dimethyl-5-methoxyoxindolylethylamine, also referred to as 3-(2-aminoethyl)-1,3-dihydro-1,3-dimethyl-5-methoxy-2H-indol-2-one, is an important intermediate in a recently discovered method of synthesizing (-)-physostigmine (B. Schonenberger and A. Brossi, Helvetica Chimica Acta 69, 1486 (1986)). While this amine can be prepared using conventional techniques, a racemic mixture is usually formed. Resolution of the racemic amine mixture into its R and S components makes it possible to synthesize (+)-physostigmine and (-)-physostigmine.

A process for the stereoselective synthesis of the amines and their precursors could provide certain advantages. Such a process could reduce or eliminate the need for resolving mixtures of enantiomers. While stereoselective syntheses that are catalyzed by enzymes are highly enantioselective, non-enzymatic processes have a wide range of selectivity. Accordingly, the results obtained in processes based on synthetic chemical techniques are generally unpredictable, and successful results in stereoselective syntheses have been difficult to achieve.

Thus, there exists a need in the art for methods based on chemical techniques for producing enantiomers of physostigmine. There also exists a need in the art for methods for the asymmetric synthesis of intermediate for use in the process. The method should make it possible to obtain the intermediates in a state of high optical purity. In addition, the process should be easy to carry out and should employ reagents that are readily available.

Summary of the invention

According, this invention aids in fulfilling these needs in the art by providing a process for the stereoselective synthesis of physostigmine of the formula

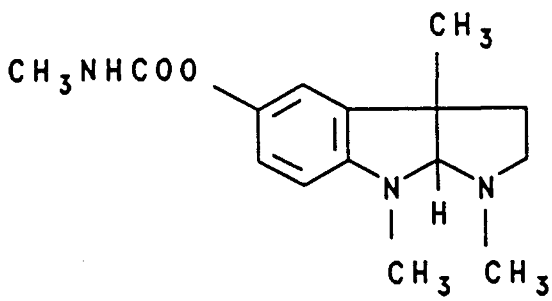

wherein the process comprises:

a) reacting a racemic oxindole of the formula

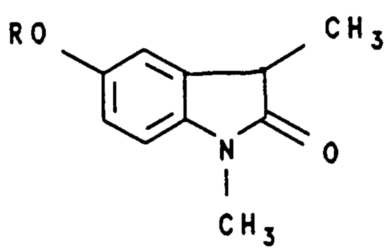

(1)

where R is selected from methyl, ethyl, and benzyl, with at least one equivalent of a halogenated acetonitrile selected from chloroacetonitrile, bromacetonitrile, and iodoacetonitrile, in a biphasic reaction mixture having an aqueous phase comprising 25 to 50 % weight of a strong inorganic base as a deprotonation agent and a solvent phase comprising an organic solvent for the oxindole selected from an aromatic hydrocarbon solvent, a halogenated aromatic solvent and a halogenated aliphatic solvent and a catalytic amount of a substituted N-benzyl cinchoninium of quinidinium compound of the formula

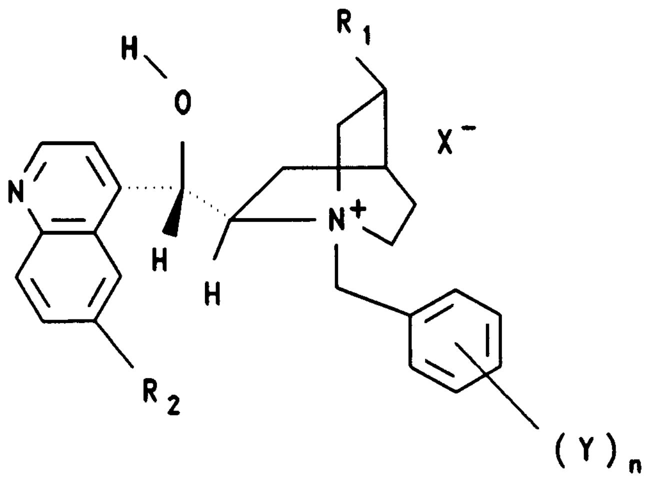

( I )

or a substituted N-benzyl cinchonidinium or quininium compound of the formula

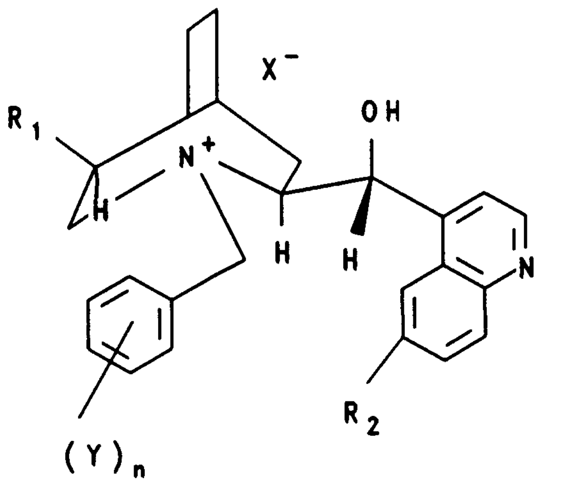

( I I )

where

- $R_1$ is a vinyl group or an ethyl group,
- $R_2$ is hydrogen or a methoxy group,
- X is chlorine or bromine,
- Y is independently selected from: hydrogen, chlorine, bromine, fluorine, trifluormethyl groups, and nitrile groups, and
- n is 1, 2, 3, 4 or 5

and agitating the biphasic reaction mixture, where the volume ratio of organic solvent phase to aqueous phase is 3:1 to 10:1, the ratio of the volume of the organic phase to the weight of the oxindole is 20:1 to 80:1, and the process is carried out at a temperature of 5°C to 30°C.

b) converting nitrile groups of the resulting alkylated oxindole to corresponding primary amines by catalytic reduction in the presence of hydrogen gas to form a mixture of enantiomers of the primary amines,

c) providing a solution of the mixture of enantiomers of the primary amines and a chiral acid with an organic solvent, wherein the chiral acid is selected from: dibenzoyl-D-tartaric acid, dibenzoyl-L-tartaric acid, ditoluoyl-D-tartaric acid and ditoluoyl-L-tartaric acid and is present in an amount sufficient to preferentially precipitate a salt of the chiral acid and one of the enantiomers, and recovering the resulting precipitate,

d) basifying the resulting tartaric acid salt of the enantiomer to form the corresponding free base, and

e) converting the resultant product into (+) or (-)-physostigmine.

Preferably, the process is used to produce (-)physostigmine from the S-form of 1,3-dimethyl-5-methoxyoxindolylethylamine.

Brief description of reaction scheme

This invention will be more fully understood by reference to the reaction scheme for the asymmetric synthesis of alkylated oxindoles 2a and 2b and conversion of these compounds to primary amines **3a** and **3b**. The primary amines are useful in the preparation of enantiomers of physostigmine and physostigmine-like compounds having pharmaceutical activity.

## DETAILED DESCRIPTION OF PREFERRED EMBODIMENTS

The asymmetric synthesis of the present invention involves conversion of an achiral substrate to a chiral product using a chiral reagent. A prochiral function serves as the precursor for a chiral product during the reaction. The following nomenclature and conventions are employed in describing this invention.

As used herein, the expression "asymmetric synthesis" means a synthesis in which an asymmetric atom, instead of being in a molecule before the commencement of the synthesis, is introduced into the molecule in the course of chemical reaction. Thus, for example, the asymmetric synthesis of the present invention is a reaction in which an achiral unit in a substrate molecule is converted by a chiral reagent into a chiral unit in such a manner that the stereoisomeric products are produced in unequal amounts.

The expression "enantioselective synthesis" means a synthesis that produces one enantiomer of a given structure in considerable predominance over the other possible enantiomer. The enantioselective synthesis of the present invention typically produces the predominant enantiomer in an amount of 70% to 90%, usually 85% to 88%, of the total enantiomers formed as products of the synthesis.

As used herein, the expressions "enantiomeric mixture" and "mixture of enantiomers" are used interchangeably to refer to racemic modifications of the enantiomers. The expressions also include solutions containing both of the enantiomers, wherein the solutions exhibit either (+) or (-) optical rotation as observed and measured with a polarimeter.

The terms "resolve" and "resolution" as used herein are intended to encompass the complete or partial separation of two enantiomers of 5-alkoxy-substituted 1,3-dimethylindolylethylamines, also referred to as 5-alkoxy substituted-3-(2-aminoethyl)-1,3-dihydro-1,3-dimethyl-2H-indol-2-one. The separation is described in more detail hereinafter. These two terms are intended to cover separations in which only one of the enantiomers is obtained in a pure state. The terms are also intended to encompass some degree of separation of the enantiomers, wherein neither of the enantiomers is obtained completely free of the other. Separation of the enantiomers may or may not be quantitative.

The heavy line in the form of a wedge

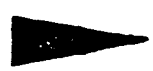

in the formulas herein signifies that the substituents are above the average plane of the ring system in connection with which the wedge appears. The heavy broken lines in the form of a wedge

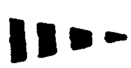

signify that the substituents are below the average plane of the ring system. For example, in the formula for one of the primary amines produced according to this invention, the methyl group in the 3-position is above the average plane of the oxindole ring, whereas the aminoethyl group is below the average plane of the ring. Thus, the methyl group and the aminoethyl group are *trans* to each other relative to the average plane of the ring.

The stereoselective synthesis of the invention can be carried out as shown in the Reaction Scheme. Referring to the Scheme an oxindole **1** can be alkylated with a halogenated acetonitrile in the presence of a chiral catalyst to give an enantiomeric mixture comprising alkylated oxindoles **2a** and **2b**, which are termed [R]- and [S]- 5-alkoxy-2,3-dihydro-1,3-dimethyl-2-oxo-1H-indole-3-acetonitriles. It was surprisingly discovered that one of the alkylated oxindoles predominates in the reaction product. In addition, it was unexpectedly found that the alkylated oxindoles **2a** and **2b** are obtained in relatively high chemical yield.

The crude enantiomeric mixture comprising the alkylated oxindoles **2a** and **2b** can be hydrogenated in the presence of a catalyst to form a mixture comprising primary amines **3a** and 3b, which are termed [R]- and [S]-5-alkoxy-3-(2-aminoethyl)-1,3-dihydro-1,3-dimethyl-2H-indol-2-ones. The primary amine 3a in which R is a methyl group is an important intermediate in the preparation of (-)-physostigmine.

The primary amine should be available in as pure a form of the optical isomer as possible in order to obtain high yields and optical purity of physostigmine. This can be achieved by selectively precipitating the enantiomer 3a or 3b with a chiral tartaric acid to form a tartaric acid salt 4a or 4b. One method for preparing the enantiomeric mixture 3a and 3b will now be described in greater detail.

The asymmetric synthesis of the present invention is carried out by the stereoselective alkylation of an oxindole of the formula:

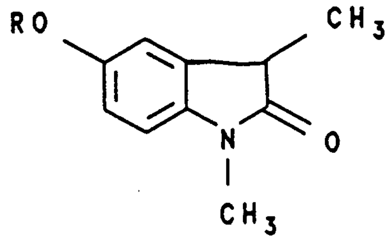

(1)

wherein the substituent R is selected from methyl (compound (1a)), ethyl (compound (1b)), and benzyl (compound (1c)). The oxindole 1 is a racemic mixture. The oxindole 1 is employed in the process of this invention as a racemic mixture, which can be prepared by the synthetic methods disclosed in F.E. King et al., J. Chem. Soc., 57:563-566 and Julian et al. J. Am. Chem. Soc. 57 (1935), 755-757 and in US-A-4,791,107.

The oxindole 1 can be selectively converted to an enantiomeric mixture comprising alkylated oxindoles 2a and 2b using a chiral phase transfer catalyst. Examples of suitable catalysts are those derived from substituted N-benzyl cinchoninium or quininium halides. The reaction is characterized by high enantioselectively.

More particularly, the stereoselective conversion of oxindole **1** to an enantiomeric mixture comprising the alkylated oxindoles **2a** and **2b** can be carried out by stirring a racemic mixture of the oxindole **1** and a chiral catalyst in a two-phase system comprised of a strong inorganic base and an organic solvent under an inert gas atmosphere until the reaction goes to substantial completion. Chemical conversion can be monitored by analyzing the reaction mixture by GLC for the formation of the alkylated oxindoles **2a** and **2b**. The enantiomer **2a** or **2b** that predominates is dependent upon the nature of the chiral catalyst that is employed.

The chiral catalyst for the selective conversion of oxindole **1** to the alkylated oxindole **2a** or **2b** is a substituted N-benzyl cinchoninium or quinidinium compound of the formula

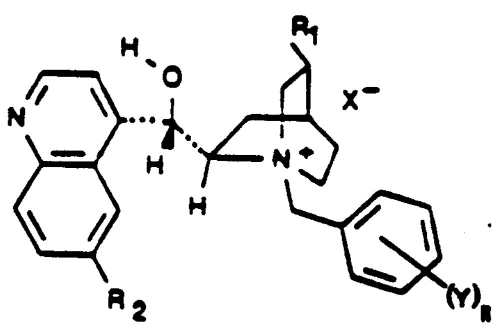

(I)

or a substituted N-benzyl cinchonidinium or quininium compound of the formula

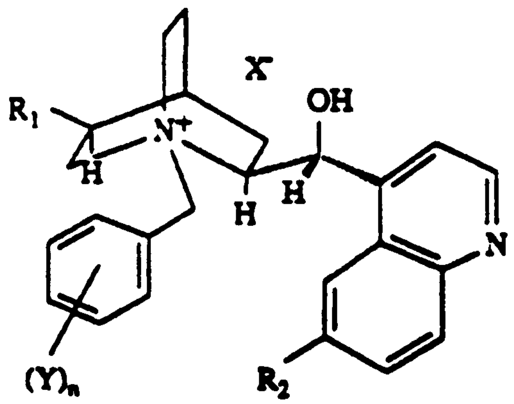

(II)

where

- R $_1$ is a vinyl group or an ethyl group,
- $R_2$ is hydrogen or a methoxy group,
- X is chlorine or bromine,
- Y is independently selected from hydrogen, chlorine, bromine, fluorine, trifluoromethyl groups, and nitrile groups; and
- n is 1, 2, 3, 4 or 5.

The substituted N-benzyl cinchoninium and the substituted N-benzyl quinidinium compounds have the formula (I) in which $R_2$ is hydrogen or methoxy, respectively. The substituted N-benzyl cinchonidinium and the substituted N-benzyl quininium compounds have the formula (II) in which $R_2$ is hydrogen or methoxy, respectively. The preferred catalysts are compounds in which Y is 3,4-dichloro or 4-trifluoromethyl. These catalysts can be prepared by utilizing the procedures described in J. Org. Chem. 1987, 52, 4745-4752 and are commercially available from Fluka Chemical Co., Hanppauge, N.Y. 11788, or from Chemical Dynamics Corporation of South Plainfield, N.J.

The substituted N-benzyl cinchoninium and quinidinium compounds and the substituted N-benzyl cinchonidinium and quininium compounds are employed in the asymmetric synthesis of the invention in an amount sufficient to catalyze the reaction of the oxindole and the halogenated acetonitrile to produce one of the enantiomers of the alkylated oxindoles in a predominant amount over the other enantiomer. For example, the catalyst can be employed in an amount of 5 to 50 mole % based upon the amount of oxindole **1**. In a preferred embodiment of this invention, the compounds are employed as catalysts in an amount of 10 to 15 mole % based upon oxindole **1**.

The substituted N-benzyl cinchoninium and quinidinium compounds provide the alkylated oxindole **2a** in excess while the substituted N-benzyl cinchonidinium and quininium compounds yield the alkylated oxindole **2b** in excess when the compounds are used in a catalytically effective amount. It will be understood that the asymmetric synthesis of this invention can also be carried out in the presence of a surfactant, such as Triton X-400. See US-A-4,578,509 and US-A-4,605,761.

Alkylation of the oxindole appears to proceed by conventional mechanisms. For this reason it was anticipated that a racemic mixture of the alkylated oxindoles would be obtained. Quite unexpectedly, however, it was found that the alkylation reaction was stereoselective and that either one of the enantiomers of the alkylated oxindoles can be obtained in excess, depending upon the choice of catalyst. Moreover, the predominant enantiomer is obtained in high chemical yield. The chemical yield is at least about 60% based on oxindole **1**, and is generally 65% to 85% based on oxindole **1**.

The stereoselective synthesis of this invention is carried out in a biphasic reaction mixture comprised of an organic solvent phase containing the racemic mixture of oxindole **1** and the catalyst and an aqueous phase containing a strong inorganic base. The oxindole **1** and the catalyst are dissolved in an aromatic hydrocarbon solvent. Halogenated aromatic solvents and halogenated aliphatic solvents can also be employed. Typical of the solvents that can be utilized are benzene, toluene, xylene, chlorobenzene, and methylene chloride. Solvent mixtures of hexane and cyclohexane can also be utilized. Technical grade solvents have been found to yield acceptable results. The preferred solvent is toluene because reaction mixtures containing this solvent gave the highest selectively of the alkylated oxindole **2a** or **2b** in the examples hereinafter. The selectivity obtained with other solvents can be optimized with a minimum of experimentation.

The aqueous phase of the reaction mixture contains a strong inorganic base, such as potassium hydroxide, sodium hydroxide, or lithium hydroxide. Technical grade bases have been found to produce acceptable results. The preferred base is sodium hydroxide because of its low cost, availability, and effectiveness in the process of the invention.

The inorganic base is employed in an amount sufficient to support catalysis of the reaction. The base functions as a deprotonation agent. It has been found that the concentration of the base in the aqueous phase affects the selectivity. The concentration of base in the aqueous phase is 25 % to 50 % by weight. As the concentration of base decreases, the selectively for one of the alkylated oxindole decreases.

The aqueous phase containing the inorganic base should have minimum solubility in the organic solvent phase containing the racemic oxindole 1 and the catalyst in order to maintain a biphasic reaction mixture. The volume ratio of the organic phase of the reaction mixture to the aqueous phase is 3:1 to 10:1. A reaction mixture containing the organic phase and the aqueous phase in a volume ratio of about 5:1 has been found to produce favorable results.

The organic solvent phase and the oxindole 1 in the reaction mixture is 20:1 to 80:1, preferably 30:1 to 45:1. The particularly preferred ratio is 40:1. These ratios are expressed as the volume of the organic solvent phase to the weight of the oxindole 1.

The alkylating agent for the racemic mixture of oxindole 1 is a halogenated acetonitrile selected from the group consisting of chloroacetonitrile, bromoacetonitrile, and iodoacetonitrile. Chloroacetonitrile is the preferred alkylating agent because it has provided the highest selectivity of the alkylated oxindoles 2a and 2b. Technical grade alkylating agents have yielded satisfactory results.

The halogenated acetonitrile is employed in an amount of at least one equivalent, and preferably 1.1 to 1.5 equivalents, of the racemic mixture of oxindole 1. Increasing the amount of the alkylating agent relative to the oxindole generally increases chemical yield, although there is no apparent advantage in utilizing the alkylating agent in large excess.

The stereoselective synthesis of the invention is carried out at a temperature of 5°C to 30°C. Lower temperatures are generally accompanied by higher selectively of the alkylated oxindole 2a or 2b, although caution must be exercised to avoid the inorganic base from separating from the aqueous solution at low temperatures. The preferred temperature range for carrying out the synthesis is 15°C to 25°C, especially about 20°C.

The stereoselective synthesis of the alkylated oxindole **2a** or **2b** is an exothermic reaction. The reaction mixture can be cooled by internal or external means to maintain the reaction temperature. The need for cooling can be minimized and even avoided by gradually adding the halogenated acetonitrile to the biphasic reaction mixture.

It is desirable to provide an inert gas blanket over the biphasic reaction mixture in which the asymmetric synthesis is carried out in order to exclude oxygen from the reaction. Examples of suitable inert gases include nitrogen, argon, and helium. Nitrogen is preferred for economic reasons.

The stereoselective synthesis of the invention can be carried out at atmospheric pressure. Sub-atmospheric pressures should be avoided.

It has been found that alkylation of the racemic mixture of oxindole **1** proceeds very rapidly. With gradual addition of the alkylating agent to the biphasic reaction mixture, the reaction is generally complete within 1 to 2 hours. Shorter reaction times can be employed, although cooling of the reaction mixture may be required. Similarly, longer reaction periods can be utilized, although there is no apparent advantage in extending the reaction time. In any event, the alkylating reaction is carried to substantial completion, which can be monitored by gas chromatography or other suitable means. In order to optimize selectivity for the alkylated oxindole **2a** or **2b**, the reaction mixture should be agitated.

The biphasic reaction mixture can be prepared as follows. The racemic mixture of oxindole **1** can be dissolved in the organic solvent and the catalyst can be added to the resulting solution. The aqueous solution of the inorganic base can then be added to the organic solution and stirred for a sufficient period to form the biphasic reaction mixture. Mild stirring for about 10 minutes has been found to be sufficient to form the biphasic mixture. The halogenated acetonitrile employed as the alkylating agent can then be added to the biphasic reaction mixture. Slow addition of the alkylating agent improves selectivity for the predominating alkylated oxindole **2a** or **2b**.

The optical purity of the enantiomers formed in the process of this invention can be expressed as the excess of the enantiomer in the reaction product as a percentage of the total enantiomers in the original solution. The amount of an enantiomer is conveniently expressed as the percent enantiomeric excess, which is abbreviated "% ee". The percent enantiomeric excess can be calculated as follows:

$$\% \text{ ee} = \frac{([A]-[B])}{([A]+[B])} \times 100$$

where

[A] is the concentration of one of the enantiomers, and
[B] is the concentration of the other enantiomer.

In a completely resolved material, the enantiomeric excess is equal in weight to the total material so that % ee, and thus optical purity, is 100%. The concentration of each of the enantiomers is, of course, expressed on the same basis, and

can be expressed on either a weight or molar basis because the enantiomers have the same molecular weight.

A number of substituted N-benzyl cinchoninium salts have been screened for selective conversion of oxindole **1** to alkylated oxindole **2a**. All the reactions were carried out by stirring a mixture of oxindole **1** (2.5 mmol) and the appropriate catalyst (0.25 mmol) in a two-phase system consisting of 8 ml of 50% NaOH and 20 ml of toluene under nitrogen for 10 min. A solution of chloroacetonitrile (2.75 mmol) in 20 ml of toluene was then added via a syringe pump over a period of 1 hour. After completion addition, the reaction mixture was analyzed by GLC for chemical conversion. Enantiomeric excess of alkylated oxindole **2a** was determined by HPLC on a Chiralcel OD column or a Chiracel OJ column (Daicel Chemical Industries Ltd.) and by NMR spectroscopy using tris[3-(heptafluoropropyl-hydroxymethylene-d-camphorato]europium (III) as the chiral shift reagent. The results are summarized in Table I.

Table I. Asymmetric Alkylation of Oxindole 1 Using Chiral Phase Transfer Catalysts.

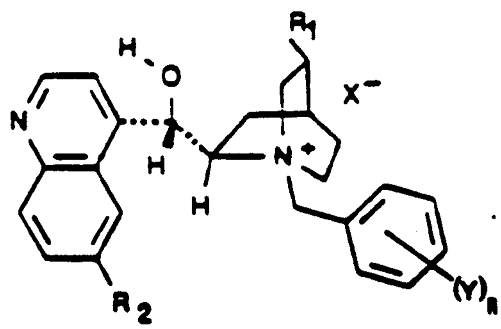

(I)

| Expts. | Catalysts | | | | % ee 2a |
|---|---|---|---|---|---|
| | R1 | R2 | Y | X | |
| 1 | vinyl | H | H | Cl | >3 |
| 2 | vinyl | H | H | Br | 10 |
| 3 | vinyl | H | 2-F | Br | 5 |
| 4 | vinyl | H | 2-$CF_3$ | Br | 4 |
| 5 | vinyl | H | 2,6-$Cl_2$ | Br | >3 |
| 6 | vinyl | H | 3-F | Br | 8 |
| 7 | vinyl | H | 3-Br | Br | 48 |
| 8 | vinyl | H | 4-Br | Br | 68 |
| 9 | vinyl | H | 4-$CF_3$* | Br | 72 |
| 10 | vinyl | H | 4-CN | Br | >2 |
| 11 | vinyl | H | 3,4-$Cl_2$ | Cl | 78 |
| 12 | vinyl | H | 3,4-$Cl_2$ | Br | 77 |
| 13** | vinyl | H | 3,4-$Cl_2$*** | Cl | 17 |
| 14**** | vinyl | H | 4-$CF_3$ | Br | 61 |
| 15 | Et | H | 4-$CF_3$ | Br | 69 |
| 16 | vinyl | $OCH_3$ | H | Br | 39 |
| 17 | vinyl | $OCH_3$ | 3,4-$Cl_2$ | Br | 77 |

*4-$CF_3$BCNB
**1:1 toluene/hexanes
***3,4-$Cl_2$-BCNC
****25% NaOH

Substitution in the 3 and/or 4 position of the benzyl moiety of the catalyst with electron withdrawing groups, such as Br, Cl, or $CF_3$, significantly increased the %ee of alkylated oxindole **2a**, (Expts. 7, 8, 9, and 12). This is probably due to a tighter ion-pair being formed as a result of increased positive character on the N-atom of the cinchoninium catalyst. That the observed enhancement of % ee by electron withdrawing groups is mainly due to inductive effect and not resonance effect is suggested by the low % ee observed for the 4-cyanobenzylcinchoninium bromide, (Expt. 10). The fluoro-substituted catalysts gave unexpectedly low % ee for reasons not yet identified, (Experiments 3 and 6). As expected, the dihydrocinchoninium catalyst behaved similarly to the corresponding cinchoninium salt, (Experiments 9

and 15). Unexpectedly, a moderate % ee was observed with benzylquinidinium bromide, (Experiment 16). No further improvement in % ee was observed when the benzyl group was further substituted with an electron withdrawing group, (Experiment 17). A slight counterion effect was observed for the case where the % ee of the reaction was low, (Experiments 1 and 2). When the % ee of the reaction was appreciably high, counterion effect was nonexistent.

It is generally not possible to separate the predominant alkylated oxindole from the other oxindole formed in the stereoselective synthesis of the invention. Therefore, the crude mixture containing the alkylated oxindole is employed in the next step of the reaction, which involves the conversion of the nitrile groups of the alkylated oxindoles to the corresponding primary amines via catalytic reduction in the presence of hydrogen gas. This step of the reaction can be carried out using conventional techniques. For example, the crude reaction product from the stereoselective synthesis can be taken up in a suitable solvent, such as methanol, ethanol, or 2-propanol. The resulting solution can be hydrogenated in the presence of a catalytic amount of metallic catalyst, such as $PtO_2$ or platinum on carbon, in an aqueous, alcoholic, concentrated HCl medium to form a mixture comprising primary amines 3a and 3b. The catalyst is typically employed in an amount of 5 % to 50 % by weight. The reaction carried out at a temperature of 15°C to 30°C for 1 hour to 2 hours until the reaction proceeds to substantial completion. Acids, such as sulfuric acid, phosphoric acid, and hydrobromic acid, can be employed in place of HCl. The % ee of the primary amines are formed in approximately the same relative proportion as that of the oxindoles at the start of the catalytic reduction of the nitrile.

The % ee of the primary amines 3a and 3b in the enantiomeric mixture from the reduction reaction is further improved by resolution with an optically active derivative of tartaric acid. Different solubility characteristics of the diastereomeric salts make it possible to preferentially isolate one of the salts. More particularly, a reaction mixture containing both of the enantiomers of the primary amine in solution is allowed to interact with an optically active derivative of tartaric acid to form a salt, which readily forms a precipitate in the reaction mixture. The enantiomer in an optically purified state is recovered from the precipitate by treatment with a mineral base.

More particularly, the enantiomers of the primary amines are resolved with a chiral acid selected from dibenzoyl-D-tartaric acid, dibenzoyl-L-tartaric acid, ditoluoyl D-tartaric acid, and ditoluoyl-L-tartaric acid. The preferred chiral acid is dibenzoyl-D-tartaric acid, because the S-enantiomer of 1,3-dimethyl-5-methoxyindolylethylamine can be selectively precipitated from an enantiomeric mixture with this acid in relatively high optical purity. It is preferred that the chiral acid be in a substantially optically pure state. The D-form of the chiral acid can be employed to preferentially precipitate enantiomer 3a, while the L-form of the chiral acid can be employed to preferentially precipitate the enantiomer 3b.

The amount of the chiral acid employed in the enrichment process will generally be 0.5 to 1 equivalent of acid per equivalent of the primary amine, and preferably 0.6 to 0.9 equivalent. It has been found that the amount of the chiral acid used as the resolving agent can affect the identity of the enantiomer of the primary amine that is preferentially precipitated. For example, when the racemic amine **3a** and **3b** is treated with one or more equivalents of dibenzoyl-D-tartaric acid in an appropriate solvent, such as acetonitrile, the diastereomeric salt corresponding to the R-enantiomer **3b** is preferentially precipitated. On the other hand, when less than 1 equivalent of dibenzoyl-D-tartaric acid is employed, the diastereomeric salt corresponding to the S-enantiomer **3a** is preferentially precipitated. In the preferred method of carrying out the enrichment process of the invention, the enantiomer **3a** is preferentially precipitated from a racemic mixture of **3a** and **3b** with dibenzoyl-D-tartaric acid in an amount of 0.6 to 0.9 equivalent of the acid per equivalent of the primary amine.

The enrichment process is carried out in a solution comprising the enantiomers and the chiral acid. The solution is prepared with an organic solvent in which the enantiomers and the chiral acid are soluble, but in which one of the tartaric acid salts of the enantiomers is less soluble so that one of the salts of the enantiomers will preferentially precipitate. The solvent is typically a liquid organic compound, such as a cyclic or acyclic substituted hydrocarbon. Ethers, such as diethyl ether, dioxane, and tetrahydrofuran, can be employed. Examples of suitable halogenated solvents are methylene chloride and chloroform. The organic compound can be an aromatic compound, such as toluene or xylene. Aliphatic nitriles, such as acetonitrile and propionitrile, can also be employed. The preferred solvent is acetonitrile.

The ratio of the solvent volume to the amount of enantiomers in the mixture being resolved can be varied over a relatively broad range. The ratio of the amount of solvent to the amount of enantiomers can typically be 5:1 to 15:1, where the ratio is expressed as the volume of solvent relative to the weight of the enantiomers in the solvent. Preferably the ratio is 8:1 to 12:1. In a preferred process of carrying out this invention, the ratio of the volume of solvent to the weight of enantiomers is about 10:1.

The solution containing the enantiomers can be prepared by dissolving the enantiomeric mixture in the solvent. Dissolution can typically be carried out at a temperature of 0°C to 60°C, but will generally be carried out at room temperature of 18°C to 22°C. Similarly, the chiral acid can be dissolved in a solvent, which is generally the same solvent as the solvent employed for the enantiomeric mixture.

After the resolving agent is added to the solution of the enantiomers, the resulting solution is aged under conditions to form a precipitate comprising a salt of the chiral acid and the enantiomer that is selectively precipitated. Aging is typically carried out at a temperature of 0°C to 30°C. The use of temperatures within the lower end of this range will generally facilitate the formation of precipitates and increase the yield because the salts are generally less soluble in the

solvent at the lower temperatures. On the other hand, the use of temperatures within the upper end of this range will generally provide higher selectivity; that is, formation of one of the salts of the enantiomers will be favored over the other salt.

Resolution of the enantiomeric mixture of the primary amines according to this invention provides a precipitate of one of the enantiomers in the form of a salt of tartaric acid. The tartaric acid salt can be converted to the corresponding free base by conventional techniques. For example, the tartaric acid salt can be dissolved in water, and the resulting solution can be treated with an aqueous solution comprised of a non-toxic inorganic base in an amount sufficient to provide a substantially basic mixture. Examples of suitable bases include sodium hydroxide, potassium hydroxide, sodium carbonate, and potassium carbonate. The amine is extracted with an organic solvent from the aqueous solution. An organic solvent, such as methylene chloride, ethyl acetate, diethyl ether, or toluene, can be employed for this purpose. The organic phase can be separated from the aqueous phase. Evaporation of the solvent from the organic phase provides the amine in the form of a free base, which can generally be utilized without further purification. Conversion of the tartaric acid salt to the corresponding free base can be carried out at ambient temperatures.

The optical purity of the primary amine **3a** or **3b** expressed as % ee obtained by the asymmetric synthesis of this invention and resolution with the optically active derivative of tartaric acid will typically be at least about 70% ee. An optical purity of 70% ee to 80% ee can be attained without further purification by recrystallization. The level of optical purity can be increased to about 96-99% ee by one or two recrystallization steps. Optimum enrichment levels can be achieved with a minimum of experimentation.

The stereoselective synthesis of the alkylated oxindoles **2a** and **2b** according to the present invention makes it possible to substantially increase the chemical yield of the enantiomer of the primary amine **3a** or **3b** of interest in the enrichment step. Specifically, enrichment of an enantiomeric mixture of the alkylated oxindoles in which one of the alkylated oxindoles predominates (as in the process of the invention) will result in a higher chemical yield of the primary amine of interest than enrichment of a racemic mixture of the alkylated oxindoles, because of the higher concentration of the desired enantiomer in the starting mixture.

The concentrations of enantiomers in a reaction mixture obtained in this invention can be determined by (1) treating the primary amine with (-)-menthyl chloroformate, followed by HPLC analysis of the corresponding diastereomeric carbamates; or (2) by treating the amine with (+)-camphorsulfonyl chloride, followed by HPLC analysis of the corresponding sulfonamide. The relative composition of a mixture of enantiomers is given by the areas under the peaks corresponding to the diastereomers in HPLC chromatograms.

The absolute configuration of the enantiomer is assigned by converting the amines to known compounds whose absolute configurations have been established. For example, the absolute configuration of the carbon atom at the 10-position of the primary amine can be determined by converting the tartaric acid salts of amines **3a** or **3b** into the corresponding optically pure primary amine **3a** or **3b** by neutralization with dilute NaOH. The resulting optically pure primary amine can be reductively cyclized in high yield by refluxing the amine in n-butanol in the presence of excess sodium metal. The product can then be derivatized with (s)-(-)-α-methylbenzyl isocyanate. The optical purity and absolute configuration of the resulting product can be confirmed by HPLC analysis according to the method of Schonenberger and Brossi, Helv. Chim. Acta., 69:1486 (1986).

This invention will be more fully understood by reference to the following examples in which all parts, proportions, ratios, and percentages are by weight unless otherwise indicated.

**Chiral Phase Transfer Alkylation**

Example 1

N-[4-(Trifluoromethyl)benzyl]cinchoninium Bromide As Catalyst

To a solution containing 0.48 g of (±)-5-methoxy-1,3 dimethyloxindole in 20 ml of toluene was added, under nitrogen, 0.13 g (10 mole %) of N-[4-(trifluoromethyl)-benzyl]cinchoninium bromide (4-$CF_3$-BCNB) followed by 8 ml of 50% NaOH. After stirring the mixture for 10 min, a solution containing 0.21 g of chloroacetonitrile in 20 ml of toluene was added dropwise over 1 hour. After complete reaction, 25 ml of ice-cold water was added. The mixture was filtered through a small celite pad rinsing with 10 ml of toluene. The filtrate was transferred to a separatory funnel, and the 2 layers were separated. The toluene extract was concentrated under reduced pressure and the residue was analyzed on a Daicel Chiralcel OD column eluting with a 10% isopropanol-hexane mixture. The enantiomeric excess of compound **2a** in which R is methyl was determined to be 72%.

Example 2

N-[3,4-(Dichloro)benzyl]cinchoninium Chloride As Catalyst

The procedure described in Example 1 was repeated with 0.12 g of N-[3,4-(dichloro)benzyl]cinchoninium chloride (3,4-$Cl_2$-BCNC) in identical fashion. The enantiomeric excess of compound **2a** in which R is methyl was found to be 78% as determined by HPLC assay of the reaction mixture.

Example 3

N-[4-Bromobenzyl]cinchoninium Bromide As Catalyst

The procedure described in Example 1 was repeated with 0.14 g of N-[4-bromobenzyl]cinchoninium bromide (4-Br-BCNB) in identical fashion. The enantiomeric excess of compound **2a** in which R is methyl was found to be 68% as determined by HPLC assay of the reaction mixture.

Example 4

N-[3-Bromobenzyl]cinchoninium Bromide As Catalyst

The procedure described in Example 1 was repeated with 0.14 g of N-[3-bromobenzyl]cinchoninium bromide (3-Br-BCNB) in identical fashion. The enantiomeric excess of compound **2a** in which R is methyl was found to be 48% as determined by HPLC assay of the reaction mixture.

Example 5

N-Benzylquinidinium Bromide As Catalyst

The procedure described in Example 1 was repeated with 0.13 g N-benzylquinidinium bromide (BQNC) in identical fashion. The enantiomeric excess of compound **2a** in which R is methyl was determined to be 39% by HPLC assay of the reaction mixture.

Example 6

N-[3,4-Dichlorobenzyl]quinidinium Chloride As Catalyst

The procedure described in Example 1 was repeated with 0.20 g of N-[3,4-dichlorobenzyl]quinidinium chloride (3,4-$Cl_2$-BQNC) in identical fashion. The enantiomeric excess of compound **2a** in which R is methyl was determined to be 77% by HPLC assay of the reaction mixture.

Example 7

N-[4-(trifluoromethyl)benzyl]dihydrocinchoninium Bromide As Catalyst

The procedure described in Example 1 was repeated with 0.13 g of N-[4-(trifluoromethyl)benzyl]dihydrocinchoninium bromide (4-$CF_3$-$H_2$-BCNB) in identical fashion. The enantiomeric excess of compound **2a** in which R is methyl was found to be 69% by HPLC assay.

Example 8

N-[4-Chlorobenzyl]cinchoninium Bromide As Catalyst

The procedure described in Example 1 was repeated with 0.13 g of N-[4-chlorobenzyl]cinchoninium bromide (4-Cl-BCNB) in identical fashion. The enantiomeric excess of compound **2a** in which R is methyl was found to be 70% by HPLC assay of the reaction mixture.

Example 9

N-[3,4-(Dichloro)benzyl]cinchoninium Bromide as Catalyst

The procedure described in Example 1 was repeated with 0.12 g of 3,4-$Cl_2$-BCNB in identical fashion. The enantiomeric excess of compound **2a** in which R is methyl was found to be 77% as determined by HPLC assay of the reaction mixture.

Example 10

Step (A): N-[3,4-(Dichloro)benzyl]cinchonium Chloride As Catalyst

To a mixture containing 5.0 g of (±)-5-methoxy-1,3 dimethyl-oxindole and 1.92 g of 3,4-$Cl_2$-BCNC (15 mole %) in 200 ml of toluene was added under an efficient $N_2$ purge 40 ml of 50% NaOH. After stirring this mixture for 10 min., a solution containing 2.17 g of choloracetonitrile in 20 ml of toluene was added over 1 hour. After complete reaction, the mixture was cooled to 10-15°C, and 160 ml of ice-cold $H_2O$ was added. The reaction mixture was filtered through a Celite pad rinsing with 40 ml of toluene. The combined filtrate was transferred to a separatory funnel, and the 2 layers were separated. The toluene solution was extracted with 100 ml of cold 3N HCl, and 100 ml of cold $H_2O$. After evaporation of solvent, 5.02 g (83%) of compound **2a** in which R is methyl was isolated as a slightly brownish oil. The enantiomeric excess of compound **2a** was determined to be 73% by HPLC.

Step (B): Catalytic Reduction of Nitriles to Primary Amines

The nitrile, **2a**, obtained from Step (A) was taken up in 50 ml of methanol and 7.25 ml of concentrated hydrochloric acid. A sample of 0.5 g of $PtO_2$ was added. The mixture was subjected to hydrogenation for 3 hours at 45 psi. The catalyst was removed by filtration through filter paper rinsing with 15 ml of methanol. The combined filtrate was concentrated under reduced pressure, and the residue was dissolved in 100 ml of ice-cold water. The acidic aqueous solution was first extracted with 50 ml of methylene chloride, and then basified with 5 ml of 50% NaOH. The basic aqueous solution was extracted with methylene chloride (3 x 50 ml). The combined organic extract was dried ($Na_2SO_4$) and concentrated under reduced pressure giving 4.70 g (92%) of the corresponding amine, **3a.**

Step (C): Enrichment of Amine by Selective Precipitation With Chiral Tartaric Acid

The amine, **3a** from Step (B) was dissolved in 25 ml of acetonitrile. A solution containing 6.42 g of dibenzoyl-D-tartaric acid in 25 ml of acetonitrile was added rapidly under nitrogen. After stirring for another 30 minutes, the precipitate that formed was filtered to give 10.38 g of a white solid. This solid was recrystallized from 60 ml of 10% water-acetonitrile mixture giving 7.86 g (47.4%) of the tartrate salt of the amine; m.p. 136-137°C. The optical purity was determined to be 99% by means of derivatization with (+)-camphorsulfonyl chloride followed by HPLC analysis of the corresponding sulfonamide.

Example 11

N-[4-(Trifluoromethyl)benzyl]cinchonidinium Bromide As Catalyst

Use of this catalyst gives predominantly the isomer leading to (+)-physostigmine.

To a stirred solution containing 1.19 g of 1,3-dimethyl-5-methoxyoxindole and 0.83 g of chloroacetonitrile in 50 ml of toluene and 10 ml of 50% NaOH under nitrogen was added 0.53 g of the above catalyst in 1 portion. After 30 min, the layers were separated. The toluene solution was washed with water, and then concentrated under reduced pressure to give the desired product in quantitative yield. The enantiomeric excess (ee) of enantiomer **2b** was determined to be 41% in the following manner. The nitrile was reduced to the corresponding amine as described in Step (B) of Example 10, followed by derivatization of the amine with (-)-menthylchloroformate with HPLC analysis of the resultant carbamate on a Whatmann Partisil PXS 10/25 column eluting with 10% acetonitrile/methylene chloride (2 ml/min; 254 nm detection).

Example 12

N-[3-(Trifluoromethyl)benzyl]cinchoninium Bromide as Catalyst

The procedure described in Example 1 was repeated with 0.13 g of N-[3-(trifluoromethyl)benzyl]cinchoninium bromide (3-$CF_3$-BCNB) in identical fashion. The enantiomeric excess of compound **2a** was found to be 68% as determined by HPLC assay of the reaction mixture.

Example 13

N-[3,4-(Dichloro)benzyl]cinchoninium Chloride as Catalyst and (+)-5-Ethoxy-1,3-dimethyloxindole as Substrate

To a mixture containing 2.15 g of (±)-5-ethoxy-1,3-dimethyl-oxindole, also referred to as 1,3-dihydro-1,3-dimethyl-5-ethoxy-2H-indol-2-one and 0.77 g of 3,4-$Cl_2$-BCNC (15 mole %) in 80 ml of toluene was added under an efficient $N_2$ purge 16 ml of 50% NaOH. After stirring this mixture for 10 min., a solution containing 0.87 g of chloroacetonitrile in 8 ml of toluene was added over 1 hour. After complete reaction, 48 ml of ice cold $H_2O$ was added. The reaction mixture was filtered through a Celite pad rinsing with 20 ml of toluene. The combined filtrate was transferred to a separatory funnel, and the two layers were separated. The toluene solution was extracted with 20 ml of 2N HCl, and twice with 20 ml of $H_2O$. After evaporation of solvent, the slightly brownish oil was assayed on a Daicel Chiralcel OD column eluting with a. 10% isopropanol-hexanes mixture. The enantiomeric excess of the compound **2a** in which R is ethyl was determined to be 71%.

Example 14

N-[3,4-(Dichloro)benzyl]cinchoninium Chloride as Catalyst and (±)-5-Benzyloxy-1,3-dimethyloxindole as Substrate

The procedure described in Example 13 was repeated with 2.80 9 of (±)-5-benzyloxy-1-1,3-dimethyloxindole, also referred to as 5-benzyloxy-1,3-dihydro-1,3-dimethyl-2H-indol-2-one in identical fashion. The enantiomeric excess of compound **2a** in which R is benzyloxy was determined to be 73% by means of HPLC assay on a Daicel Chiralcel OJ column eluting with 40% isopropanol-hexanes.

The compound (±)-5-methoxy-1,3-dimethyl-oxindole employed in the Examples is also referred to as 1,3-dihydro-1,3-dimethyl-5-methoxy-2H-indol-2-one.

The process of this invention has a number of advantages. The process for the stereoselective synthesis of enantiomers provides precursors of physostigmine in high chemical yield and purity. The availability of one enantiomer of a given structure in considerable predominance over other enantiomers makes it possible to enhance the results obtained when the enantiomers are subsequently resolved. The techniques for carrying out the stereoselective synthesis do not present any unusual difficulties. The reagents required for the process are readily available or can be easily prepared using conventional techniques. This invention provides a practical, economical process for the total synthesis of selected enantiomers of physostigmine.

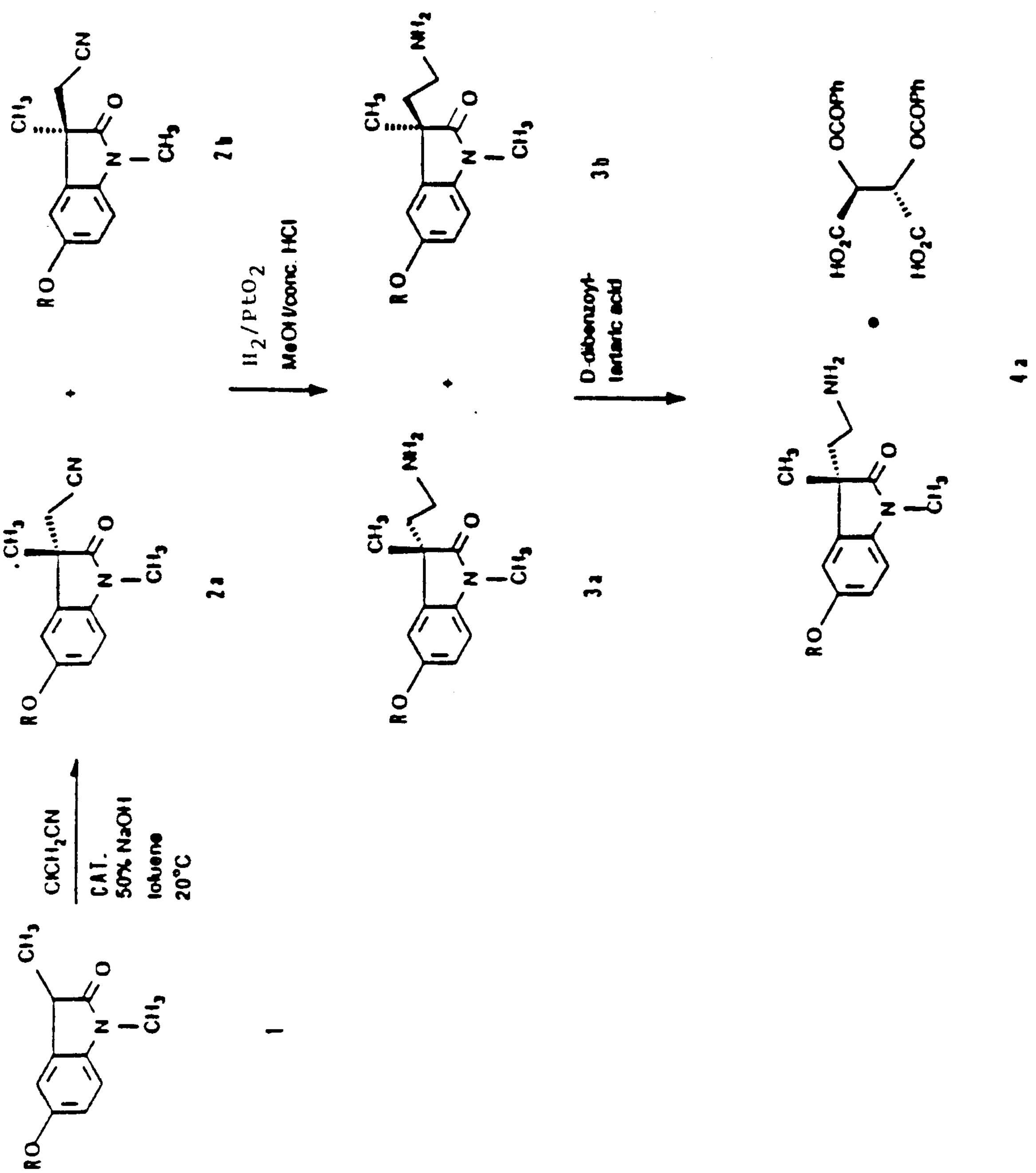


**Claims**

1. A process for the stereoselective synthesis of physostigmine of the formula

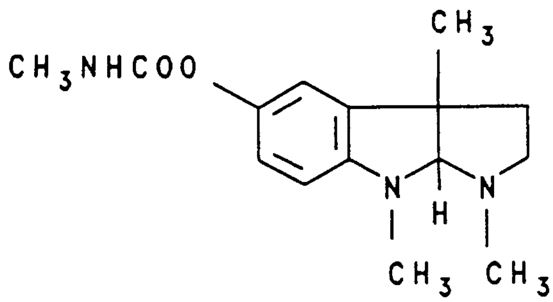

wherein the process comprises:

a) reacting a racemic oxindole of the formula

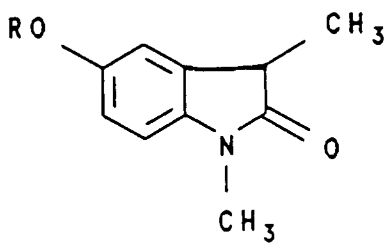

(1)

where R is selected from methyl, ethyl, and benzyl, with at least one equivalent of a halogenated acetonitrile selected from chloroacetonitrile, bromacetonitrile, and iodoacetonitrile, in a biphasic reaction mixture having an aqueous phase comprising 25 to 50 % weight of a strong inorganic base as a deprotonation agent and a solvent phase comprising an organic solvent for the oxindole selected from an aromatic hydrocarbon solvent, a halogenated aromatic solvent and a halogenated aliphatic solvent and a catalytic amount of a substituted N-benzyl cinchoninium or quinidinium compound of the formula

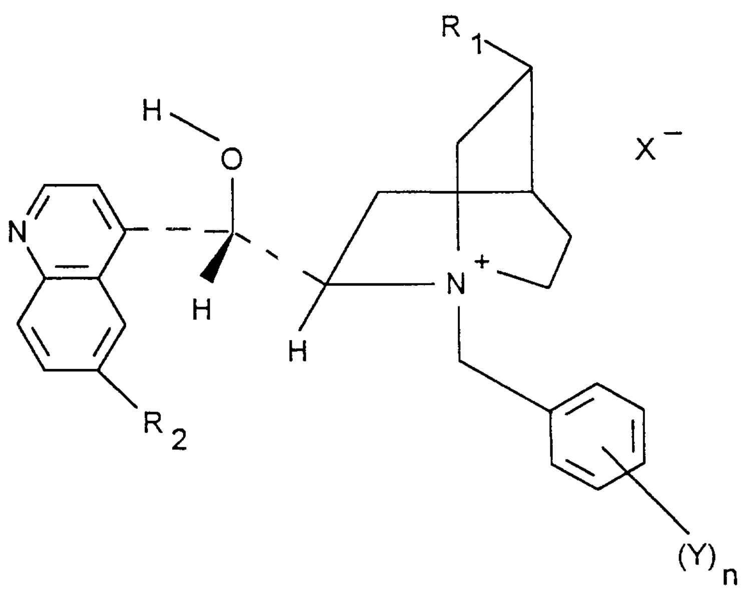

(I)

or a substituted N-benzyl cinchonidinium or quininium compound of the formula

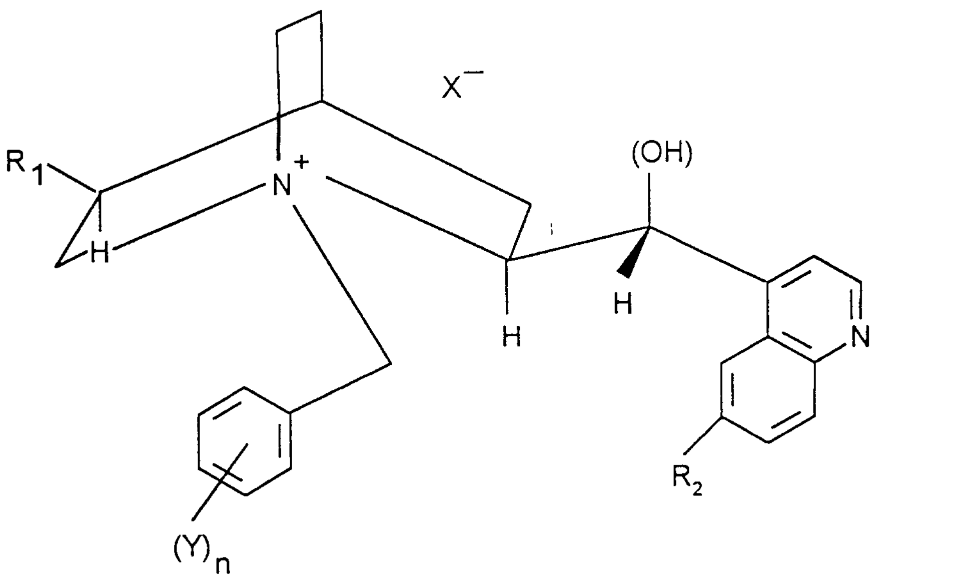

(II)

where

- $R_1$ is a vinyl group or an ethyl group,
- $R_2$ is hydrogen or a methoxy group,
- X is chlorine or bromine,
- Y is independently selected from: hydrogen, chlorine, bromine, fluorine, trifluormethyl groups, and nitrile groups, and
- n is 1, 2, 3, 4, or 5

and agitating the biphasic reaction mixture, where the volume ratio of organic solvent phase to aqueous phase is 3:1 to 10:1, the ratio of the volume of the organic solvent phase to the weight of the oxindole is 20:1 to 80:1, and the process is carried out at a temperature of 5°C to 30°C,

b) converting nitrile groups of the resulting alkylated oxindole to corresponding primary amines by catalytic reduction in the presence of hydrogen gas to form a mixture of enantiomers of the primary amines,

c) providing a solution of the mixture of enantiomers of the primary amines and a chiral acid with an organic solvent wherein the chiral acid is selected from: dibenzoyl-D-tartaric acid, dibenzoyl-L-tartaric acid, ditoluoyl-D-tartaric acid and ditoluoyl-L-tartaric acid and is present in an amount sufficient to preferentially precipitate a salt of the chiral acid and one of the enantiomers, and recovering the resulting precipitate,

d) basifying the resulting tartaric acid salt of the enantiomer to form the corresponding free base, and

e) converting the resultant product into (+) or (-)-physostigmine.

**2.** Process as claimed in claim 1, wherein the halogenated acetonitrile is 1.1 to 1.5 equivalents based on the oxindole.

**3.** Process as claimed in claim 1 or 2, wherein the halogenated acetonitrile is chloroacetonitrile.

**4.** Process as claimed in any of one of the preceding claims, wherein the stereoselective synthesis is carried out in an inert gas atmosphere.

**5.** Process as claimed in claim 1, wherein substantially all of the oxindole is reacted with the halogenated acetonitrile.

**6.** Process as claimed in any of the preceding claims, wherein the biphasic reaction mixture is stirred.

**7.** Process as claimed in any one of the preceding claims, wherein R is methyl and Y is 3,4-dichloro or 4-trifluormethyl.

8. Process as claimed in claim 1, wherein the solvent is selected from: benzene, toluene, xylene, chlorobenzene, methylene chloride, and a mixture of one of the above solvents with hexane or cyclohexane.

9. Process as claimed in claim 1, wherein the organic solvent phase and the aqueous phase in the biphasic reaction mixture are in a volume ratio of 5:1.

10. Process as claimed in claim 1, wherein the solvent phase and the racemic oxindole are in a ratio of about 30:1 to about 45:1 (v/w).

11. Process as claimed in claim (1), wherein in step c the ratio of the volume of solvent to the total weight of enantiomers of the primary amines is 8:1 to 12:1.

12. Process as claimed in claim 11, wherein the ratio of the volume of solvent to the total weight of enantiomers of the primary amines is 10:1.

13. Process is claimed in claim 1, wherein in step c the solvent is acetonitrile.

14. Process as claimed in claim I(c), wherein the chiral acid is employed in an amount of 0.5 to 1 equivalents of acid per equivalent of enantiomers of the primary amines.

**Patentansprüche**

1. Verfahren zur stereoselektiven Synthese von Physostigmin der Formel

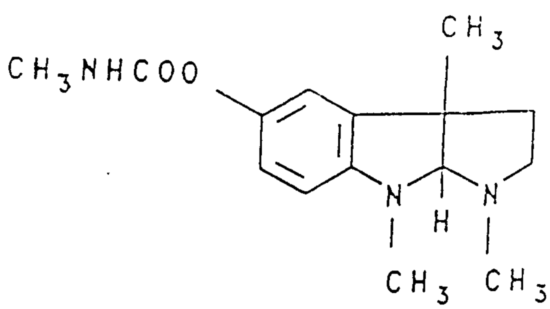

wobei der Prozeß folgende Schritte umfaßt:

a) Umsetzen eines racemischen Oxindols der Formel

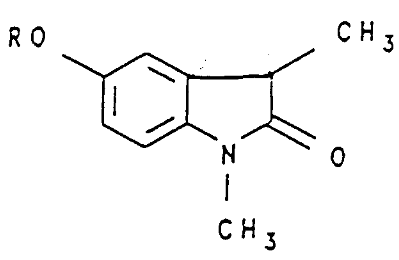

(1)

wobei R aus der Gruppe Methyl, Ethyl und Benzyl ausgewählt wird, mit mindestens einem Äquivalent eines halogenierten Acetonitrils, das unter Chloracetonitril, Bromacetonitril und Jodacetonitril ausgewählt wird, in einem biphasischen Reaktionsgemisch mit einer wäßrigen Phase aus 25 bis 50 Gew.-% einer starken anorganischen Base als Deprotonierungsagens und einer Lösemittelphase, umfassend ein organisches Lösemittel für das Oxindol, ausgewählt aus einem aromatischen Kohlenwasserstoff-Lösemittel, einem halogenierten aromatischen Lösemittel und einem halogenierten aliphatischen Lösemittel, und einer katalytischen Menge einer substituierten N-Benzylcinchoninium- oder -chinidiniumverbindung der Formel

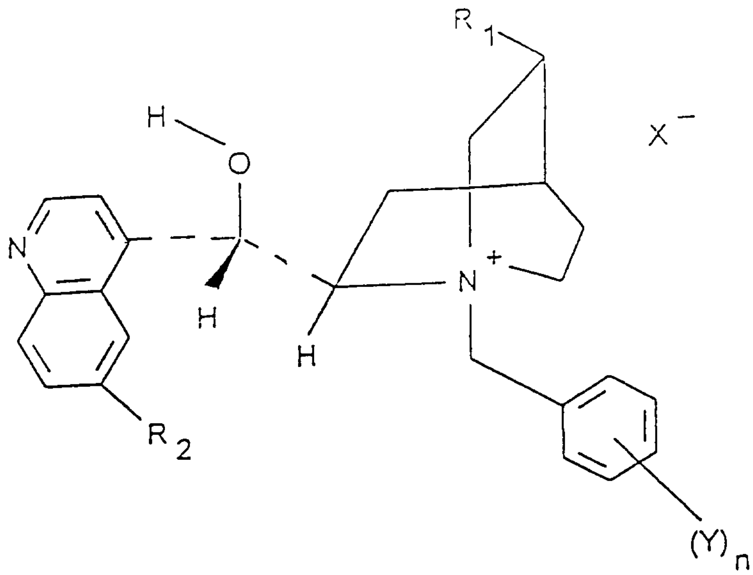

(I)

oder einer substituierten N-Benzylcinchonidinium- oder -chiniumverbindung der Formel

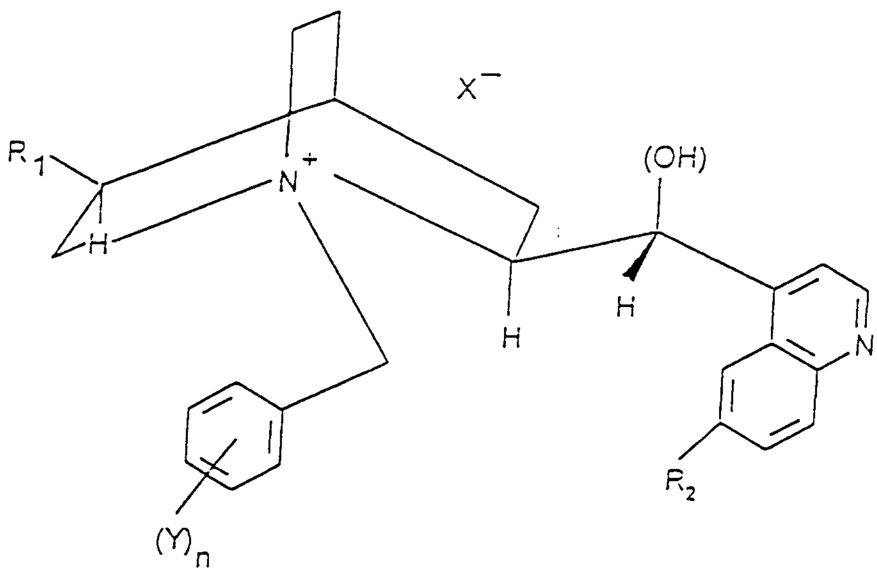

(II)

wobei

$R_1$ eine Vinylgruppe oder eine Ethylgruppe ist,

$R_2$ Wasserstoff oder eine Methoxygruppe ist,

X Chlor oder Brom ist,

Y unabhängig ausgewählt wird aus Wasserstoff, Chlor, Brom, Fluor, Trifluormethylgruppen und Nitrilgruppen, und

n 1, 2, 3, 4 oder 5 ist,

und Rühren des biphasischen Reaktionsgemischs, wobei das Volumenverhältnis der organischen Lösemittelphase und der wäßrigen Phase 3:1 bis 10:1 beträgt, das Verhältnis der organischen Lösemittelphase und des

Oxindolgewichts (V/G) 20:1 bis 80:1 beträgt und das Verfahren bei einer Temperatur von 5°C bis 30°C durchgeführt wird,

b) Umsetzen der Nitrilgruppen des entstehenden alkylierten Oxindols zu entsprechenden primären Aminen durch katalytische Reduktion in Gegenwart von Wasserstoffgas, um ein Gemisch von Enantiomeren der primären Amine zu erhalten,

c) Herstellen einer Lösung des Gemischs von Enantiomeren der primären Amine und einer chiralen Säure mit einem organischen Lösemittel, wobei die chirale Säure ausgewählt wird aus: Dibenzoyl-D-weinsäure, Dibenzoyl-L-weinsäure, Ditoluoyl-D-weinsäure und Ditoluoyl-L-weinsäure und in einer ausreichenden Menge vorhanden ist, um vorzugsweise ein Salz der chiralen Säure und einem der Enantiomeren zu fällen, und Aufnehmen des entstehenden Präzipitats,

d) basische Einstellung des entstehenden Weinsäuresalzes des Enantiomeren, um die entsprechende freie Base herzustellen, und

e) Umsetzen des entstehenden Produkts zu (+) oder (-)-Physostigmin.

**2.** Verfahren gemäß Anspruch 1, wobei das halogenierte Acetonitril gegenüber dem Oxindol 1,1 bis 1,5 Äquivalenten entspricht.

**3.** Verfahren gemäß Anspruch 1 oder 2, wobei das halogenierte Acetonitril Chloracetonitril ist.

**4.** Verfahren gemäß einem der vorstehenden Ansprüche, wobei die stereoselektive Synthese unter Inertgas durchgeführt wird.

**5.** Verfahren gemäß Anspruch 1, wobei im wesentlichen das gesamte Oxindol mit dem halogenierten Acetonitril umgesetzt wird.

**6.** Verfahren gemäß einem der vorstehenden Ansprüche, wobei das biphasische Reaktionsgemisch gerührt wird.

**7.** Verfahren gemäß einem der vorstehenden Ansprüche, wobei R für Methyl und Y für 3,4-Dichlor oder 4-Trifluormethyl steht.

**8.** Verfahren gemäß Anspruch 1, wobei das Lösemittel ausgewählt wird aus Benzol, Toluol, Xylol, Chlorbenzol, Methylenchlorid und einem Gemisch eines der vorstehenden Lösemittel mit Hexan oder Cyclohexan.

**9.** Verfahren gemäß Anspruch 1, wobei die organische Lösemittelphase und die wäßrige Phase im biphasischen Reaktionsgemisch in einem Volumenverhältnis von 5:1 vorhanden sind.

**10.** Verfahren gemäß Anspruch 1, wobei die Lösemittelphase und das racemische Oxindol in einem Verhältnis von ca. 30:1 bis ca. 45:1 (V/G) vorliegen.

**11.** Verfahren gemäß Anspruch 1, wobei in Schritt (c) das Verhältnis des Volumens des Lösemittels und des Gesamtgewichts der Enantiomeren der primären Amine von 8:1 bis 12:1 beträgt.

**12.** Verfahren gemäß Anspruch 11, wobei das Volumen des Lösemittels zum Gesamtgewicht der Enantiomeren der primären Amine 10:1 beträgt.

**13.** Verfahren gemäß Anspruch 1, wobei in Schritt (c) das Lösemittel Acetonitril ist.

**14.** Verfahren gemäß Anspruch 1 (c), wobei die chirale Säure in einer Menge von 0,5 bis 1 Äquivalenten Säure pro Äquivalent Enantiomere der primären Amine eingesetzt wird.

## Revendications

**1.** Procédé pour la synthèse stéréosélective de la physostigmine de formule

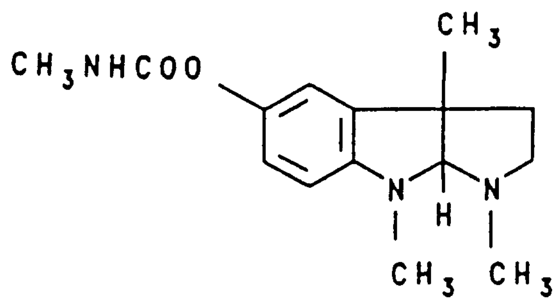

le procédé comprenant:

a) la mise en réaction d'un oxindole racémique de formule

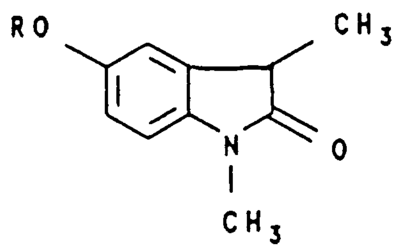

( 1 )

dans laquelle R est choisi parmi les groupes méthyle, éthyle et benzyle, avec au moins 1 équivalent d'un acétonitrile halogéné choisi parmi le chloracétonitrile, le bromoacétonitrile et l'iodoacétonitrile, dans un mélange réactionnel en deux phases comportant une phase aqueuse comprenant de 25 à 50 % en poids d'une base minérale forte en tant qu'agent de déprotonation et une phase de solvant comprenant un solvant organique pour l'oxindole, choisi parmi un solvant de type hydrocarbure aromatique, un solvant aromatique halogéné et un solvant aliphatique halogéné, et une quantité catalytique d'un composé de type N-benzylcinchonium ou -quinidinium substitué de formule

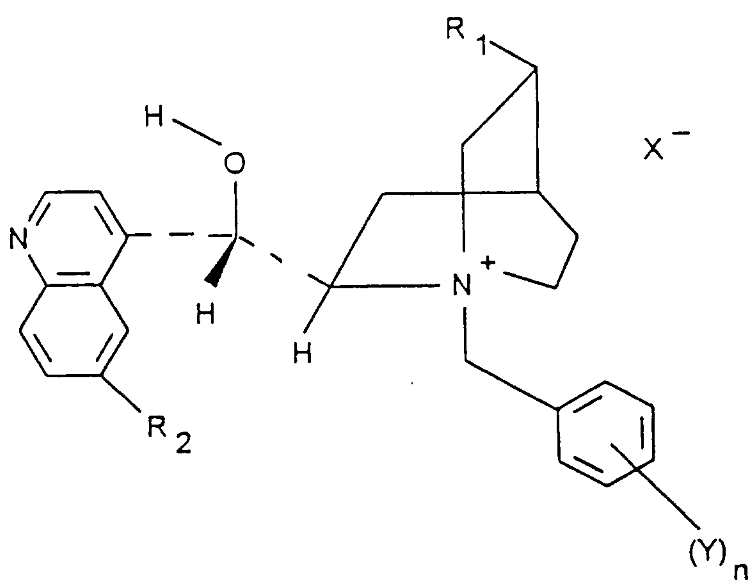

(I)

ou un composé de type N-benzylcinchonidinium ou -quininium de formule

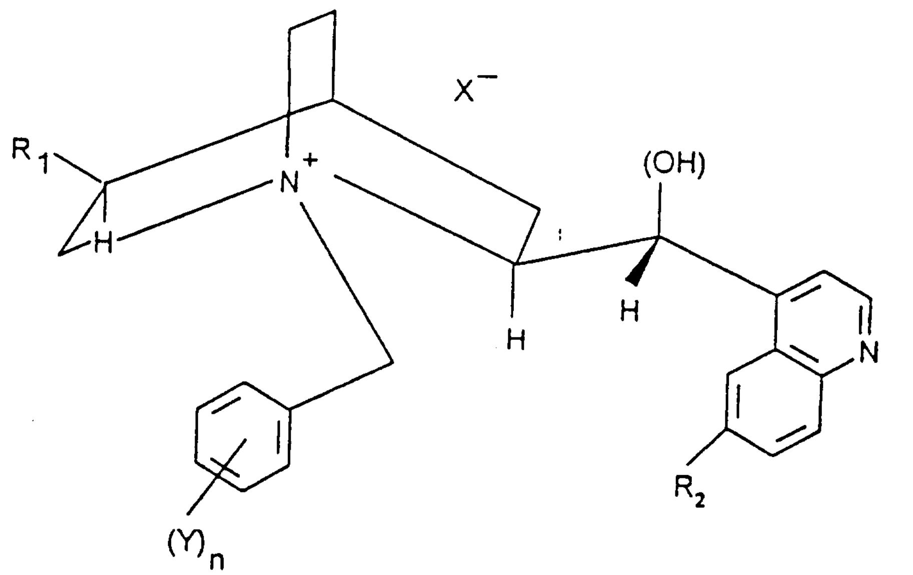

(II)

formules dans lesquelles

- $R_1$ est le groupe vinyle ou éthyle,
- $R_2$ est un atome d'hydrogène ou le groupe méthoxy,
- X est le chlore ou le brome,
- Y est choisi indépendamment parmi un atome d'hydrogène, de chlore, brome, fluor et les groupes trifluorométhyle et nitrile, et
- n est 1, 2, 3, 4 ou 5,

et l'agitation du mélange réactionnel en deux phases, le rapport en volume de la phase de solvant organique à la phase aqueuse allant de 3:1 à 10:1, le rapport du volume de la phase de solvant organique au poids de l'oxindole allant de 20:1 à 80:1, et le procédé étant effectué à une température de 5 à 30°C,
b) la conversion des groupes nitrile de l'oxindole alkylé résultant en groupes amino primaires correspondants par réduction catalytique en présence d'hydrogène gazeux, pour la formation d'un mélange d'énantiomères des amines primaires,
c) la préparation d'une solution du mélange d'énantiomères des amines primaires et d'un acide chiral avec un solvant organique, l'acide chiral étant choisi parmi l'acide dibenzoyl-D-tartrique, l'acide dibenzoyl-L-tartrique, l'acide ditoluyl-D-tartrique et l'acide ditoluyl-L-tartrique et étant présent en une quantité suffisante pour faire précipiter préférentiellement un sel de l'acide chiral et de l'un des énantiomères, et la récupération du précipité résultant,
d) l'alcalinisation du sel résultant de l'énantiomère avec l'acide tartrique, pour la formation de la base libre correspondante, et
e) la conversion du produit résultant en (+) ou (-)-physostigmine.

2. Procédé selon la revendication 1, dans lequel l'acétonitrile halogéné est utilisé à raison de 1,1 à 1,5 équivalent par rapport à l'oxindole.

3. Procédé selon la revendication 1 ou 2, dans lequel l'acétonitrile halogéné est le chloracétonitrile.

4. Procédé selon l'une quelconque des revendications précédentes, dans lequel la synthèse stéréosélective est effectuée dans une atmosphère constituée par un gaz inerte.

5. Procédé selon la revendication 1, dans lequel pratiquement la totalité de l'oxindole est mise en réaction avec l'acétonitrile halogéné.

6. Procédé selon l'une quelconque des revendications précédentes, dans lequel le mélange réactionnel en deux phases est agité.

**7.** Procédé selon l'une quelconque des revendications précédentes, dans lequel R est le groupe méthyle et Y est le groupe 3,4-dichloro ou 4-trifluorométhyle.

**8.** Procédé selon la revendication 1, dans lequel le solvant est choisi parmi le benzène, le toluène, le xylène, le chlorobenzène, le chlorure de méthylène et un mélange de l'un des solvants ci-dessus avec l'hexane ou le cyclohexane.

**9.** Procédé selon la revendication 1, dans lequel la phase de solvant organique et la phase aqueuse dans le mélange réactionnel en deux phases sont en un rapport en volume de 5:1.

**10.** Procédé selon la revendication 1, dans lequel la phase de solvant et l'oxindole racémique sont en un rapport d'environ 30:1 à environ 45:1 (v/p).

**11.** Procédé selon la revendication 1, dans lequel, dans l'étape c, le rapport du volume du solvant au poids total des énantiomères des amines primaires va de 8:1 à 12:1.

**12.** Procédé selon la revendication 11, dans lequel le rapport du volume du solvant au poids total des énantiomères des amines primaires est 10:1.

**13.** Procédé selon la revendication 1, dans lequel, dans l'étape c, le solvant est l'acétonitrile.

**14.** Procédé selon la revendication 1(c), dans lequel l'acide chiral est utilisé en une quantité de 0,5 à 1 équivalent d'acide par équivalent d'énantiomères des amines primaires.